# EUROPEAN PATENT SPECIFICATION

(11) **EP 3 622 994 B1**
(45) Date of publication and mention of the grant of the patent: **26.05.2021**
(21) Application number: 18194746.6
(22) Date of filing: 17.09.2018
(51) Int. Cl.: A61M 16/06, A61M 16/08

(54) **RESPIRATORY MASK WITH IMPROVED HEADGEAR CONNECTING SYSTEM**
ATEMMASKE MIT VERBESSERTEM KOPFBANDVERBINDUNGSSYSTEM
MASQUE RESPIRATOIRE COMPORTANT UN SYSTÈME DE CONNEXION DE HARNAIS AMÉLIORÉ

(43) Date of publication of application: 18.03.2020
(73) Proprietor: Air Liquide Medical Systems, 92160 Antony (FR)
(72) Inventor: MASSERDOTTI, Fulvio, 25073 Bovezzo (IT); ALBERICI, Luca, 25073 Bovezzo (IT); MASSARO, Paolo, 25073 Bovezzo (IT)
(74) Representative: Air Liquide

(56) References cited:
- WO-A1-2012/067523
- WO-A1-2014/015382
- WO-A1-2017/042717
- US-A1- 2016 074 614

## Description

The present invention concerns a respiratory mask, especially a nasal pillow mask, comprising a mask body and a headgear detachably attached to the mask body, and respiratory gas delivering assembly comprising a gas source, preferably a CPAP or BPAP device, and such a respiratory mask.

Respiratory masks are commonly used for treating sleep apnea or other respiratory troubles or diseases. A respiratory mask comprises a mask body, also called a "frame" or "shell", a flexible cushion fixed to the mask body and that comes into contact with the face of the patient, and a headgear, also called a "harness", comprising straps for maintaining the mask in position on the face of a patient, while the mask is used.

Different kinds of masks exist, such as nasal masks covering only the nose, facial mask or full-face masks covering the nose and the mouth, nasal pillow masks comprising prongs inserted into the nostrils... Examples for respiratory masks, especially nasal pillow masks, are given by WO-A-2017/042717, WO-A-2014/077708, EP-A-2679267, US-A-6431172, US-A-2009/032026, US-A-2011/209709 and US-A-2010/108073. WO2014015382A1 discloses the features the preamble of claim 1.

Despite the fact that various connecting systems have been proposed for fixing of the headgear to the mask body, such as hook/loop or slot/loop systems, snap-fit connectors..., the fixing of the headgear to the mask remains a problem for many patients.

Indeed, many patients encounter difficulties in fixing the headgear to the mask, because they cannot see what they are doing, once they have put the mask in position against their face. They hence often waste a significant amount of time, when trying to connect the headgear to the mask body, and, in many cases, require the assistance of another person, or of extra material, such as a mirror or the like.

Further, although Velcro-type ribbon connections may be easier to attach, they are often not tight enough for avoiding disconnection, due to the motions of the patient overnight.

A goal of the present invention is to propose a respiratory mask comprising an improved connecting system allowing an easy fixing and securing of the headgear to the mask body, and ensuring that the headgear is not easily detached, despite uncontrolled motions of the user, i.e. a patient, overnight.

The solution of the present invention concerns a respiratory mask, especially a nasal pillow mask, comprising a mask body comprising elongated arms projecting away from the mask body, said elongated arms and said mask body being made in one-piece, and a headgear detachably secured to said elongated arms. The headgear is used for fixing and maintaining the mask on the face of the user, i.e. the patient.

According to the present invention, as defined in claim 1, at least one of said elongated arms comprises at least one pin element, protruding from an outer surface of said elongated arm, and the headgear comprises at least one flexible sleeve structure comprising at least one traversing hole, so that said at least one of the elongated arms is at least partially inserted into said at least one flexible sleeve structure, and said at least one pin element is inserted into said at least one traversing hole, for thereby securing the headgear to the mask body.

A respiratory mask according to the present invention can further comprise one or more of the following additional features.
- the respiratory mask comprises two elongated arms, each comprising at least one pin element, i.e. one or several pin elements.
- the elongated arms of the mask body cooperate with the flexible sleeve structure of the headgear, whereas the pin elements cooperate with the traversing holes, for securing the headgear to the mask body.
- the elongated arms have preferably a ribbon-like structure, i.e. a flat elongated structure.
- each elongated arm has a length of between 5 cm and 30 cm, typically of less than about 20 cm.
- each elongated arm has a width of between 0,5 cm and 5 cm, typically of less than about 4 cm.
- each elongated arm comprises at least two opposite surfaces comprising an inner surface and an outer surface, the inner surface being arranged toward the face of the patient, when the patient carries the mask, preferably at least a part of the inner surface comes into contact with the face of the patient, in use, typically with the cheek regions of the patient.
- the two elongated arms are at least partially inserted into the two flexible sleeve structures, i.e. a first elongated arm, for instance a right elongated arm, is at least partially inserted into a first flexible sleeve structure, whereas a second elongated arm, for instance a left elongated arm is at least partially inserted into a second flexible sleeve structure.
- the headgear comprises two flexible sleeve structures each comprising at least one traversing hole, i.e. one or several traversing holes.
- the headgear comprises two flexible sleeve structures that are symmetrically-arranged.
- each flexible sleeve structure has a length of between 5 cm and 30 cm.
- each flexible sleeve structure comprises at least one traversing hole having a size (e.g. diameter) of between 0,1 mm and 30 mm, preferably of less than 20 mm.
- the headgear further comprises flexible straps.
- the headgear further comprises flexible straps forming a loop-structure, i.e. a ring-like structure.
- the flexible sleeve structures are attached to the flexible straps, for instance stitched, glued, thermos-welded....
- the flexible straps and the flexible sleeve structures are made of a woven material, i.e. woven fibers or fabric material.
- the woven material comprises a polymer fabric, such as UBL Lycra Polyurethane.
- the flexible sleeve structures are (slightly) stretchable.
- the headgear comprises flexible straps made of woven polymer material or the like.
- the pin elements carried by the two elongated arms are inserted into the traversing holes arranged into the two flexible sleeve structures.
- each pin element protrudes from an outer surface of each elongated arm.
- each pin element has a height of between 0,1 and 30 mm, preferably of less than 15 mm.
- each pin element comprises an enlarged head, preferably the enlarged head is supported by a pillar- or leg-structure.
- each pillar- or leg-structure supporting an enlarged head is integrally-arranged on an elongated arm, preferably carried by or fixed to the outer surface of said elongated arm.
- each pin element has a picot-like structure or shape.
- a pin element is retained in a traversing hole by means of its enlarged head.
- the elongated arms and/or the pin elements are made in one piece of a polymer material, preferably as polycarbonate (PC), polypropylene (PP), polyamide (PA), acrylonitrile butadiene styrene (ABS) or polystyrene (PS).
- the respiratory mask further comprises a flexible cushion secured to the mask body, preferably the flexible cushion is detachable, i.e. can be removed and replaced or cleaned, for instance when worn out or dirty.
- the flexible cushion is made of silicone or the like.
- the flexible cushion comprises two hollow prongs (i.e. little hollow conducts) for delivering a respiratory gas, such as air or oxygen-enriched air, to the nostrils of the patient.
- the flexible cushion comprises two hollow prongs that are sized and configured for entering/be inserted into the nostrils of the patient, when said patient wears the mask.
- the mask body is rigid.
- the mask body is rigid and made of plastic material, such as polycarbonate, polypropylene, acrylonitrile butadiene styrene, polyamide, polystyrene or the like.
- the mask comprises a plane of symmetry, i.e. it has a symmetrical structure or shape.
- the flexible cushion is detachably fixed to the mask body by a snap-fit connection or the like.
- the two elongated arms project laterally on each side of the mask body, i.e. left and right sides (i.e. with respect to the plane of symmetry of the mask).
- each elongated arm comprises a proximal end fixed to the mask body and a free distal end.
- the two elongated arms and the mask body are made in one piece, preferably molded in one-piece.
- the two hollow prongs are in fluid communication with the inner gas chamber of the mask body.
- each hollow prong comprises a truncated cone-shaped free end.
- the hollow prongs are flexible.
- the mask body further comprises a gas inlet, such as a central opening.
- the mask body further comprises a hollow elbow connector fluidly connected to the gas inlet.
   - the mask body further comprises an inner gas chamber or compartment.
- the hollow elbow connector is in fluid communication with the inner gas chamber, through the gas inlet, for delivering the respiratory gas to said inner gas chamber, i.e. for allowing gas travelling into the lumen of the elbow connector entering into the gas chamber.
- the hollow elbow connector further comprises one or several venting ports for venting to the atmosphere CO₂-enriched gases expired by the patient.
- said one or several venting ports, such as a large opening or venting port, used for venting to the atmosphere CO₂-enriched gases expired by the patient, is covered (i.e. closed) by a gas-permeable membrane element, such as a woven or fabric mesh material (i.e. woven fibers) or the like.
- the hollow elbow connector is fluidly connected to the downstream end of a flexible hose (i.e. tube or conduct) so that the gas circulating into said flexible hose can be delivered to the lumen of the hollow elbow connector.
- the flexible hose is made of plastic material.
- the flexible hose further comprises an upstream end in fluid communication with a hollow swivel connector.
- the respiratory mask is a nasal pillow mask.

Furthermore, the invention also concerns a respiratory gas delivering assembly comprising:
- a gas source, preferably a CPAP or BPAP device, delivering a pressurized respiratory gas, such as air under pressure (i.e. pressure > 1 cm H₂O), and
- a respiratory mask according to the present invention, preferably a nasal pillow mask comprising two hollow prongs entering/inserted into the nostrils of the patient, when said patient wears the mask.

Further, a flexible hose, such as a plastic tube or the like, is used for conveying the gas delivered by the gas source to the respiratory mask thereby delivering said gas to the airways of the patient. Preferably, the flexible hose is fluidly connected to a hollow connector, preferably an elbow connector, fixed to the mask body.

Other features, aspects and advantages of the present invention will become apparent from the following detailed description when taken in conjunction with the accompanying drawings which illustrates, by way of examples, the present invention, among which:
- Figure 1 shows an embodiment of a respiratory mask, namely a nasal pillow mask, according to the present invention,
- Figures 2 and 3 show the mask body of the mask of Figure 1,
- Figures 4 and 5 are different views of a portion of a headgear for the mask of Figure 1, and
- Figures 6 and 7 illustrate the securing of the headgear to the mask body of the mask of Figure 1.

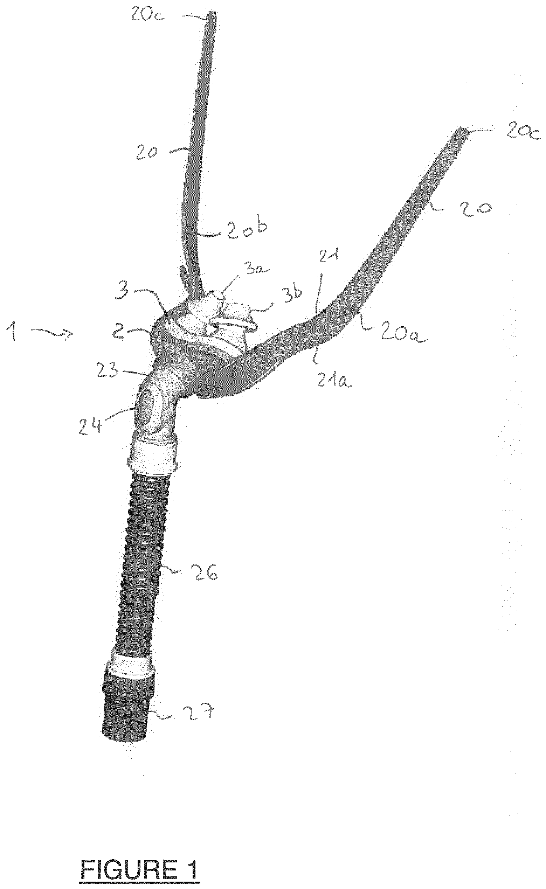

Figure 1 shows an embodiment of a respiratory mask 1 according to the present invention, namely a nasal pillow mask, that comprises a flexible cushion 3 detachably fixed to a rigid mask body 2, also called the mask "frame" or "shell". The mask 1 has a symmetrical general shape, i.e. comprises a (vertically-oriented) plane of symmetry.

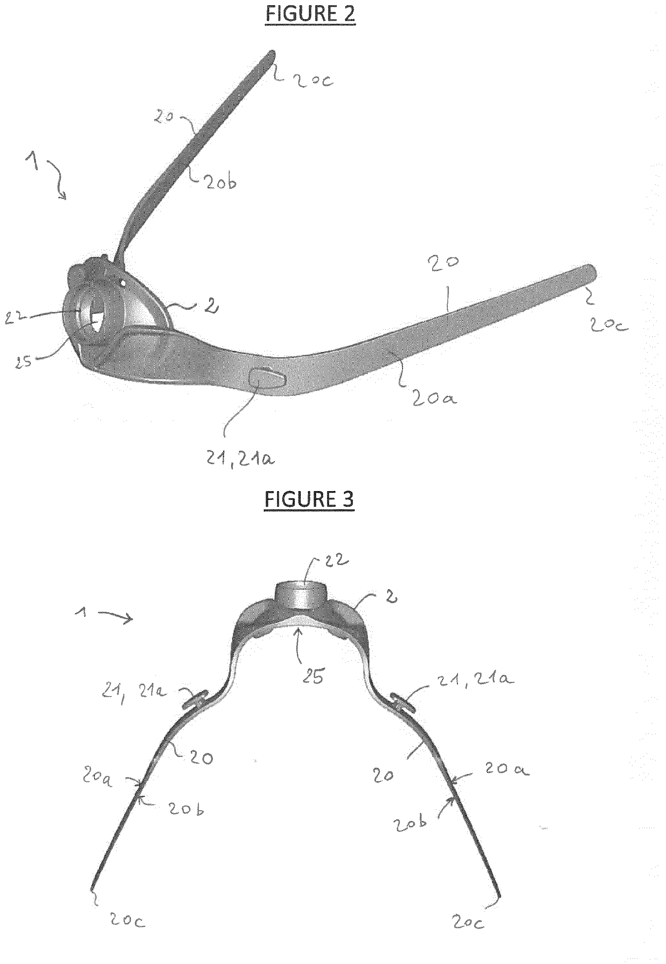

As shown in Figures 2 and 3, the mask body 2 comprises two elongated arms 20 laterally-projecting on each side of the mask body 2, i.e. left and right sides (with respect to the plane of symmetry of the mask).

The two lateral arms 20 have an elongated flat structure, such as a ribbon-like structure, and each comprise two opposite surfaces, namely an outer surface 20a and an inner surface 20b. The inner surface 20b is in contact with the face of the patient, when he/she wears the mask 1, in particular with his/her cheek regions, whereas the outer surface 20a is externally-oriented.

The two lateral arms 20 and the mask body 2 are integral, preferably they are molded in one-piece, for instance made of a plastic material.

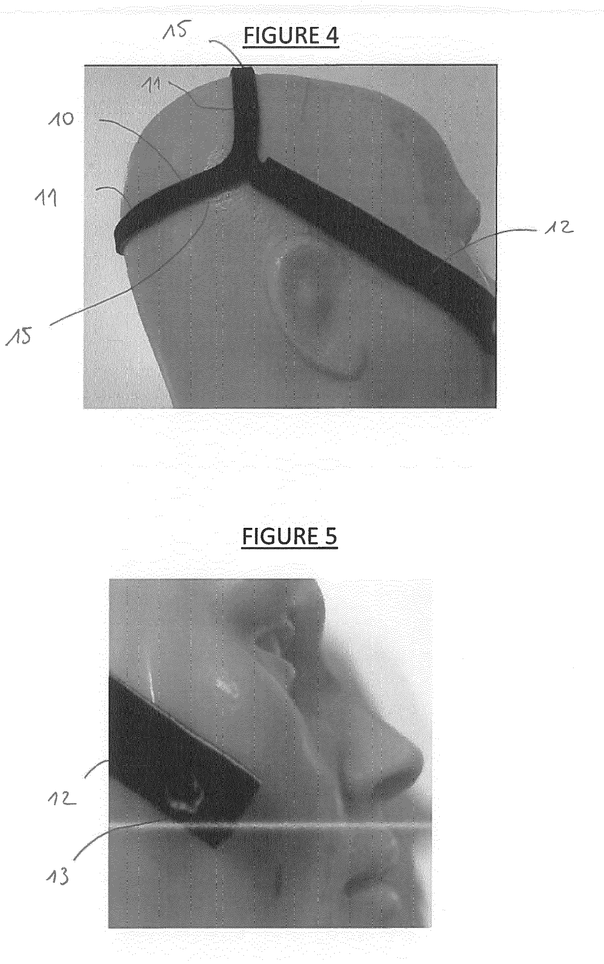

Further, a headgear 10 (shown in Figures 4-6), also called a harness, is used for fixing and maintaining the mask, in use, on the face of the user, i.e. the patient. The headgear 10 is secured to the lateral arms 20 of the mask body 2 by means of an improved connecting system, as below detailed.

The headgear 10 comprises one or several straps 11 made of fabric, preferably of a polymer woven material or the like. As visible in Fig. 4, said straps 11 form a loop or ring-structure 15, i.e. it comprises a "O"-shape structure, that comes into contact with the top of the patient's head for well-maintaining the headgear in position, when the patient wears the mask 1, especially overnight.

The flexible cushion 3 is made for instance of silicone, whereas the mask body 2 and the two lateral arms 20 are made of plastic material, such as polycarbonate, polypropylene, acrylonitrile butadiene styrene, polyamide or polystyrene. Further, the flexible cushion 3 comprises two flexible hollow prongs 3a, 3b for delivering the respiratory gas (e.g., air, O₂-enriched air...) to the nostrils of the patient. In use, the free enlarged heads of the prongs 3a, 3b are inserted into the nostrils of the patient.

Further, as shown in Fig. 1-3, the mask body 2 comprises a gas inlet 22, i.e. central opening, that receives a hollow tubular connector 23 that conveys into its lumen and delivers a respiratory gas, such as air.

The mask body 2 and the flexible cushion 3 delimit an inner gas chamber 25 or compartment that receives the respiratory gas circulating into the lumen of the hollow tubular connector 23. In other words, the gas inlet 22 and the two hollow prongs 23a, 23b of the flexible cushion 23 are in fluid communication with the inner gas chamber 25.

Preferably, the flexible cushion 3 comprises an inner annular rim comprising a rigid ring secured thereto, for instance snap-fitted, over-molded, glued or thermo-welded together, so that said rigid ring is sandwiched between the flexible cushion 3 and the mask body 2, when they are assembled together, thereby providing a tight and efficient coupling of the cushion 3 to the mask body 2. The rigid ring provides an increased rigidity to the flexible cushion 3 that eases its coupling to the mask body 2.

According to the present invention, the headgear 10 is detachably secured to the two elongated arms 20 that project away from the mask body 2, i.e. laterally-arranged on the right and left sides of the mask body 2, by means of an improved coupling system.

More precisely, the coupling system of the invention comprises one or several pin elements 21 arranged on at least one of the two elongated arms 20, preferably at least one pin element 21 arranged on each elongated arms 20, that cooperate(s) with one or several flexible sleeve structures 12 of the headgear 10, that each comprises at least one traversing hole 13.

Preferably, each elongated arm 20 comprises (at least) one pin element 21 arranged on its outer surface 20a, whereas the headgear 10 comprises two flexible sleeve structures 12, each comprising (at least) one traversing hole 13.

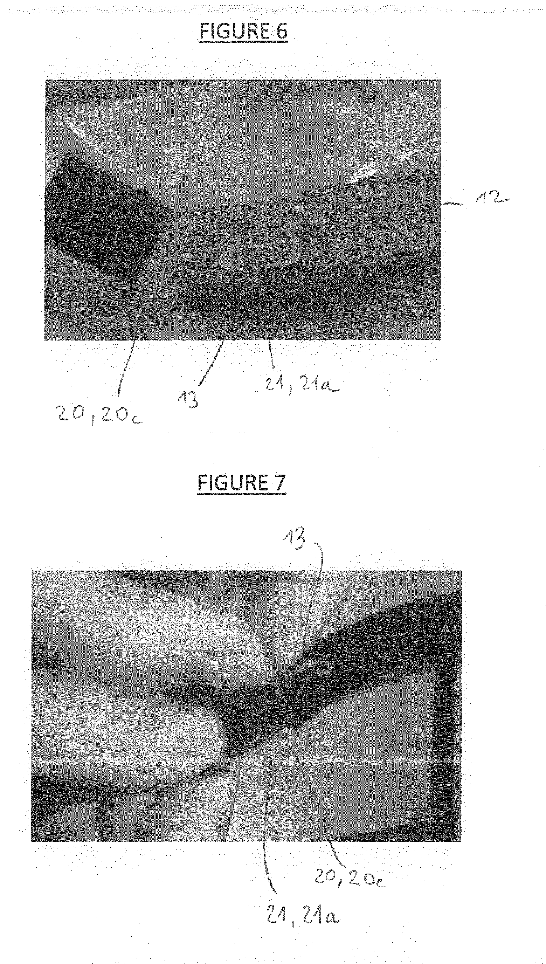

As illustrated in Fig. 6 and 7, for coupling the headgear 10 to the mask body 2, the free end 20c of each elongated arm 20 is progressively inserted by the user, i.e. the patient, into the lumen of a corresponding flexible sleeve structure 12, until when each pin element 21 can be inserted into a corresponding traversing hole 13, thereby securing the headgear 10 to the mask body 2.

As visible on Fig. 3, 6 and 7, the pin elements 21 comprise an enlarged head 21a arranged on a pillar-like element attached to the outer surface 20a of the elongated arms 20. The enlarged heads 21a help retaining and maintaining the pin elements 21 into their corresponding traversing holes 3.

The coupling system of the present invention leads to a better coupling and safer attachment of the headgear to the mask body, compared to masks of the prior art, avoiding undesired detachments, due to patients' motions overnight.

Further, the coupling system of the present invention also allows an easy adjustment of the headgear to the size and morphology of the patient's head, thanks to the use of a resilient material, i.e. elastic material, that is able to be slightly stretched. This avoids using complex mechanisms that are complicated for the patients.

The flexible sleeve structure 12 of the headgear 10 are fixed to the straps 11, especially to the "O"-shape structure, as shown in Fig. 4. For instance, they are stitched, glued, thermo-welded... together. Preferably, the flexible sleeve structure 12 are also made of a fabric material, preferably of a polymer woven material or the like.

Furthermore, a hollow connector 23, preferably an elbow connector, comprising one or several venting ports 24, is fixed to the gas inlet 22 of the mask body 2 for providing gas to the inner gas chamber 25. The venting ports 24 are used for venting to the atmosphere, the CO₂-enriched gases expired by the patient. The hollow elbow connector 23 is itself fluidly connected to the downstream end of a flexible hose 26 (i.e. tube) so that the respiratory gas conveyed by said flexible hose 26 can be provided to the lumen of the connector 23. The flexible hose 26 further comprises an upstream end in fluid communication with a hollow swivel connector 27 that can be connected to a source of respiratory gas, such as CPAP or BPAP device, delivering a pressurized respiratory gas, such as air under pressure (i.e. pressure > 1 cm H₂O).

The respiratory mask 1 according to the present invention is useable for treating respiratory failures or disorders, such as sleep apnea or the like.

## Claims

1. Respiratory mask (1) comprising a mask body (2) comprising elongated arms (20) projecting away from the mask body (2), said elongated arms and said mask body (2) being made in one-piece, and a headgear (10) detachably secured to said elongated arms (20), each elongated arm (20) comprising a proximal end fixed to the mask body (2) and a free distal end (20c), the headgear (10) further comprising at least one flexible sleeve structure (12) comprising at least one traversing hole (13), said at least one elongated arms (20) is at least partially inserted into said at least one flexible sleeve structure (12),
**characterized in that** said at least one of the elongated arms (20) comprises at least one pin element (21) protruding from an outer surface (20a) of said elongated arm (20), said at least one pin element (21) being inserted into said at least one traversing hole (13).

2. Respiratory mask according to the preceding claim, **characterized in that** it comprises:
- two elongated arms (20) each comprising at least one pin element (21), and
- the headgear (10) comprises two flexible sleeve structures (12) each comprising at least one traversing hole (13),
wherein said elongated arms (20) are at least partially inserted into said flexible sleeve structures (12), and said pin elements (21) are inserted into said traversing holes (13).

3. Respiratory mask according to any one of the preceding claims, **characterized in that** it further comprises a flexible cushion (3) secured to the mask body (2), preferably the flexible cushion (3) is made of silicone.

4. Respiratory mask according to any one of the preceding claims, **characterized in that** the headgear (10) further comprises flexible straps (11), said flexible sleeve structures (12) being attached to the flexible straps (11).

5. Respiratory mask according to any one of the preceding claims, **characterized in that** the flexible straps (11) and the flexible sleeve structures (12) are made of a woven material.

6. Respiratory mask according to claim 5, **characterized in that** the woven material comprises a polymer fabric.

7. Respiratory mask according to any one of the preceding claims, **characterized in that** the elongated arms (20) have a ribbon-like structure.

8. Respiratory mask according to any one of the preceding claims, **characterized in that** each pin element (21) has a picot-like structure.

9. Respiratory mask according to any one of the preceding claims, **characterized in that** each pin element (21) comprises an enlarged head (21a).

10. Respiratory mask according to any one of the preceding claims, **characterized in that** the pin element (21) is retained in a traversing hole (13) by means of its enlarged head (21a).

11. Respiratory mask according to anyone of the preceding claims, **characterized in that** the elongated arms (20) and the pin elements (21) are made in one piece of a polymer material, preferably polycarbonate (PC), polypropylene (PP), polyamide (PA), acrylonitrile butadiene styrene (ABS) or polystyrene (PS).

12. Respiratory mask according to any one of the preceding claims, **characterized in that** the flexible cushion (3) comprises two hollow prongs.

13. Respiratory mask according to any one of the preceding claims, **characterized in that** the mask body (2) further comprises a gas inlet (22).

14. Respiratory mask according to any one of the preceding claims, **characterized in that** the mask body (2) further comprises a hollow elbow connector (23) fluidly connected to the gas inlet (22).

15. Respiratory gas delivering assembly comprising:
- a gas source, preferably a CPAP or BPAP device, delivering a pressurized respiratory gas, such as air under pressure, and
- a respiratory mask (1) according to any one of the preceding claims, preferably a nasal pillow mask comprising two hollow prongs (3a, 3b) inserted into the nostrils of the patient, when said patient wears the mask (1).

## Patentansprüche

1. Atemmaske (1), umfassend einen Maskenkörper (2), der längliche Arme (20) umfasst, die von dem Maskenkörper (2) wegragen, wobei die länglichen Arme und der Maskenkörper (2) einteilig hergestellt sind, und ein Kopfband (10), das lösbar an den länglichen Armen (20) befestigt ist, wobei jeder längliche Arm (20) ein an dem Maskenkörper (2) befestigtes proximales Ende und ein freies distales Ende (20c) umfasst, wobei das Kopfband (10) ferner mindestens eine flexible Hülsenstruktur (12) umfasst, die mindestens ein Durchgangsloch (13) umfasst, wobei der mindestens eine längliche Arm (20) mindestens teilweise in die mindestens eine flexible Hülsenstruktur (12) eingeführt wird,
**dadurch gekennzeichnet, dass** der mindestens eine der länglichen Arme (20) mindestens ein Stiftelement (21) umfasst, das von einer Außenfläche (20a) des länglichen Arms (20) vorragt, wobei das mindestens eine Stiftelement (21) in das mindestens eine Durchgangsloch (13) eingeführt wird.

2. Atemmaske nach dem vorhergehenden Anspruch, **dadurch gekennzeichnet, dass** sie Folgendes umfasst:
- zwei längliche Arme (20), die jeweils mindestens ein Stiftelement (21) umfassen, und
- das Kopfband (10) umfasst zwei flexible Hülsenstrukturen (12), die jeweils mindestens ein Durchgangsloch (13) umfassen,
wobei die länglichen Arme (20) mindestens teilweise in die flexiblen Hülsenstrukturen (12) eingeführt werden und die Stiftelemente (21) in die Durchgangslöcher (13) eingeführt werden.

3. Atemmaske nach einem der vorhergehenden Ansprüche, **dadurch gekennzeichnet, dass** sie ferner ein flexibles Kissen (3) umfasst, das an dem Maskenkörper (2) befestigt ist, wobei das flexible Kissen (3) vorzugsweise aus Silikon hergestellt ist.

4. Atemmaske nach einem der vorhergehenden Ansprüche, **dadurch gekennzeichnet, dass** das Kopfband (10) ferner flexible Gurte (11) umfasst, wobei die flexiblen Hülsenstrukturen (12) an den flexiblen Gurten (11) angebracht sind.

5. Atemmaske nach einem der vorhergehenden Ansprüche, **dadurch gekennzeichnet, dass** die flexiblen Gurte (11) und die flexiblen Hülsenstrukturen (12) aus einem gewebten Material hergestellt sind.

6. Atemmaske nach Anspruch 5, **dadurch gekennzeichnet, dass** das gewebte Material ein Polymergewebe umfasst.

7. Atemmaske nach einem der vorhergehenden Ansprüche, **dadurch gekennzeichnet, dass** die länglichen Arme (20) eine bandartige Struktur haben.

8. Atemmaske nach einem der vorhergehenden Ansprüche, **dadurch gekennzeichnet, dass** jedes Stiftelement (21) eine picot-artige Struktur hat.

9. Atemmaske nach einem der vorhergehenden Ansprüche, **dadurch gekennzeichnet, dass** jedes Stiftelement (21) einen vergrößerten Kopf (21a) umfasst.

10. Atemmaske nach einem der vorhergehenden Ansprüche, **dadurch gekennzeichnet, dass** das Stiftelement (21) mittels seines vergrößerten Kopfs (21a) in einem Durchgangsloch (13) gehalten wird.

11. Atemmaske nach einem der vorhergehenden Ansprüche, **dadurch gekennzeichnet, dass** die länglichen Arme (20) und die Stiftelemente (21) einteilig aus einem Polymermaterial, vorzugsweise Polycarbonat (PC), Polypropylen (PP), Polyamid (PA), Acrylnitril-Butadien-Styrol (ABS) oder Polystyrol (PS), hergestellt sind.

12. Atemmaske nach einem der vorhergehenden Ansprüche, **dadurch gekennzeichnet, dass** das flexible Kissen (3) zwei hohle Ausläufer umfasst.

13. Atemmaske nach einem der vorhergehenden Ansprüche, **dadurch gekennzeichnet, dass** der Maskenkörper (2) ferner einen Gaseinlass (22) umfasst.

14. Atemmaske nach einem der vorhergehenden Ansprüche, **dadurch gekennzeichnet, dass** der Maskenkörper (2) ferner einen hohlen Winkelverbinder (23) umfasst, der fluidisch mit dem Gaseinlass (22) verbunden ist.

15. Atemgaszufuhranordnung, umfassend:
- eine Gasquelle, vorzugsweise eine CPAP- oder eine BPAP-Vorrichtung, die ein unter Druck stehendes Atemgas wie Druckluft zuführt, und
- eine Atemmaske (1) nach einem der vorhergehenden Ansprüche, vorzugsweise eine Nasen-Kissen-Maske, die zwei hohle Ausläufer (3a, 3b) umfasst, die in die Nasenlöcher des Patienten eingeführt sind, wenn der Patient die Maske (1) trägt.

## Revendications

1. Masque respiratoire (1) comprenant un corps de masque (2) comprenant des bras allongés (20) faisant saillie à partir du corps de masque (2), lesdits bras allongés et ledit corps de masque (2) étant réalisés en une seule pièce, et un harnais (10) fixé de manière détachable auxdits bras allongés (20), chaque bras allongé (20) comprenant une extrémité proximale fixée au corps de masque (2) et une extrémité distale libre (20c), le harnais (10) comprenant en outre au moins une structure de manchon flexible (12) comprenant au moins un trou traversant (13), ledit au moins un bras allongé (20) étant au moins partiellement inséré dans ladite au moins une structure de manchon flexible (12),
**caractérisé en ce que** ledit au moins un des bras allongés (20) comprend au moins un élément de broche (21) faisant saillie depuis une surface extérieure (20a) dudit bras allongé (20), ledit au moins un élément de broche (21) étant inséré dans ledit au moins un trou traversant (13).

2. Masque respiratoire selon la revendication précédente, **caractérisé en ce qu'**il comprend :
- deux bras allongés (20) comprenant chacun au moins un élément de broche (21), et
- le harnais (10) comprend deux structures de manchon souple (12) comprenant chacune au moins un trou traversant (13),
lesdits bras allongés (20) étant au moins partiellement insérés dans lesdites structures de manchon flexible (12), et lesdits éléments de broche (21) étant insérés dans lesdits trous traversants (13).

3. Masque respiratoire selon l'une quelconque des revendications précédentes, **caractérisé en ce qu'**il comprend en outre un coussin souple (3) fixé au corps de masque (2), de préférence le coussin souple (3) est en silicone.

4. Masque respiratoire selon l'une quelconque des revendications précédentes, **caractérisé en ce que** le harnais (10) comprend en outre des sangles souples (11), lesdites structures de manchons souples (12) étant attachées aux sangles souples (11).

5. Masque respiratoire selon l'une quelconque des revendications précédentes, **caractérisé en ce que** les sangles souples (11) et les structures de manchons souples (12) sont réalisées dans un matériau tissé.

6. Masque respiratoire selon la revendication 5, **caractérisé en ce que** le matériau tissé comprend un tissu polymère.

7. Masque respiratoire selon l'une quelconque des revendications précédentes, **caractérisé en ce que** les bras allongés (20) ont une structure en ruban.

8. Masque respiratoire selon l'une quelconque des revendications précédentes, **caractérisé en ce que** chaque élément de broche (21) a une structure de type picot.

9. Masque respiratoire selon l'une quelconque des revendications précédentes, **caractérisé en ce que** chaque élément de broche (21) comprend une tête élargie (21a).

10. Masque respiratoire selon l'une quelconque des revendications précédentes, **caractérisé en ce que** l'élément de broche (21) est retenu dans un trou traversant (13) au moyen de sa tête élargie (21a).

11. Masque respiratoire selon l'une quelconque des revendications précédentes, **caractérisé en ce que** les bras allongés (20) et les éléments de broche (21) sont réalisés d'un seul tenant dans un matériau polymère, de préférence en polycarbonate (PC), en polypropylène (PP), en polyamide (PA), en acrylonitrile butadiène styrène (ABS) ou en polystyrène (PS).

12. Masque respiratoire selon l'une quelconque des revendications précédentes, **caractérisé en ce que** le coussin souple (3) comprend deux branches creuses.

13. Masque respiratoire selon l'une quelconque des revendications précédentes, **caractérisé en ce que** le corps de masque (2) comprend en outre une entrée de gaz (22).

14. Masque respiratoire selon l'une quelconque des revendications précédentes, **caractérisé en ce que** le corps de masque (2) comprend en outre un raccord coudé creux (23) relié fluidiquement à l'entrée de gaz (22).

15. Ensemble de délivrance de gaz respiratoire comprenant :
- une source de gaz, de préférence un dispositif CPAP ou BPAP, délivrant un gaz respiratoire sous pression, tel que de l'air sous pression, et
- un masque respiratoire (1) selon l'une quelconque des revendications précédentes, de préférence un masque à coussin nasal comprenant deux branches creuses (3a, 3b) insérées dans les narines du patient, lorsque ledit patient porte le masque (1).
